# EUROPEAN PATENT SPECIFICATION

(11) **EP 3 988 055 B1**
(45) Date of publication and mention of the grant of the patent: **05.06.2024**
(21) Application number: 21806112.5
(22) Date of filing: 18.01.2021
(51) Int. Cl.: A61F 2/24, A61M 39/22

(54) **TRICUSPID VALVE PROSTHESIS**
TRIKUSPIDALE KLAPPENPROTHESE
PROTHÈSE DE VALVE TRICUSPIDE

(30) Priority: 25.08.2020 CN 202010862817; 25.08.2020 CN 202021793124 U
(43) Date of publication of application: 27.04.2022
(73) Proprietor: Jiangsu Trulive Medtech Co., Ltd, Rudong County Nantong Jiangsu 226400 (CN)
(72) Inventor: WEI, Lixuan, Shanghai 201206 (CN)
(74) Representative: Argyma
(86) International application number: PCT/CN2021/072486
(87) International publication number: WO 2022/041638

(56) References cited:
- WO-A1-2016/186909
- WO-A1-2018/077145
- CN-A- 102 256 568
- CN-A- 102 438 546
- CN-A- 102 438 546
- CN-A- 111 904 664
- US-A1- 2005 222 488
- US-A1- 2009 264 997
- US-A1- 2011 245 911
- US-A1- 2013 030 521
- US-A1- 2016 089 238
- US-A1- 2018 014 931

## Description

### BACKGROUND OF THE INVENTION

### Field of the Invention

The present invention relates to the technical field of medical instruments, in particular, to a valve prosthesis implanted into a heart for replacing a native tricuspid valve.

### Description of the Prior Art

A heart valve is a membrane-like structure that can open and close inside an organ of a person or some animals. Everyone has four valves in his/her heart. The four valves are an aortic valve linking a left ventricle with an aorta, a pulmonary valve linking a right ventricle with a pulmonary artery, a mitral valve linking a left atrium with the left ventricle, and a tricuspid valve linking a right atrium with the right ventricle. They all function as a "one-way valve" so that the blood can only flows from one direction to the other direction without flowing reversely.

With the development of social economy and the aging of population, the incidence of valvular heart disease has increased significantly. Studies have shown that the incidence of valvular heart disease in the population over 75 years of age is as high as 13.3%. At present, traditional surgical treatment is still the preferred treatment for patients with severe valvular lesions. However, for the older patients with combined multi-organ disease, a history of thoracotomy, and poor heart function, the traditional surgery has disadvantages of high risk and mortality and even no access to surgery for some patients.

As an atrioventricular valve of the right heart, the tricuspid valve has a structure similar to that of the mitral valve and includes the valve leaflets, the valve annulus, the chordae tendineae, the papillary muscle, and the cardiac muscle. The transcatheter tricuspid valve replacement (TTVR) or the transcatheter tricuspid valve implantation (TTVI) has advantages of no need for thoracotomy, of small trauma and of quick recovery for patients. Therefore, TTVR or TTVI has been widely concerned by experts and scholars.

Although the technique of tricuspid valve replacement develops rapidly, there are still some recognized challenges in the design of the valve. For example, one of the challenges might be anchoring for the valve. The existing design for the tricuspid valve basically performs anchoring by clamping the valve leaflets or capturing the valve leaflets. These two ways of anchoring will stretch the chordae tendineae, causing damages on the native valve leaflets. It has also been suggested that using the interatrial septum for anchoring. However, this way of anchoring is not secure and further needs to clamp the valve leaflets additionally, which otherwise may easily cause falling-off and produce safety hazards. For example, another one of the challenges might be the risk of conduction block. A stent body performs the anchoring while the stent may compress the conduction tissues, causing the risk of the conduction block. Document US2016089238A1 describes a valve prosthesis. US2016089238A1 does not disclose that the anchoring structure is disposed above the stent body for anchoring the stent body at the native valve annulus for preventing the stent body from displacing. US2016089238A1 does not disclose that the anchoring structure is configured to be at least partly attached to the fossa ovalis of the interatrial septum to form a retention force by being attached to the fossa ovalis so that the anchoring effect on the valve prosthesis is achieved. US2016089238A1 does not disclose that the anchoring structure comprises a first anchoring member configured to be provided with at least one protruding portion, and the protruding portion is embedded into the fossa ovalis for the anchoring effect.

Also document WO2018077145A1 describes a tricuspid valve prosthesis.

### SUMMARY OF THE INVENTION

The invention provides a tricuspid valve prosthesis, which may solve the above drawbacks in the prior art.

The technical solution of the invention is as follows:
A tricuspid valve prosthesis includes a stent body implanted at a tricuspid valve annulus for supporting the prosthetic leaflets, and an anchoring structure disposed above the stent body for anchoring the stent body at a native valve annulus for preventing the stent body from displacing, wherein the anchoring structure is configured to be partially attached to a fossa ovalis of an interatrial septum to form a retention force by being attached to the fossa ovalis so that an anchoring effect on the valve prosthesis is achieved. Inspired by the native structure of the fossa ovalis, the invention clamps and fixes the anchoring structure in the fossa ovalis partially by making full use of a depression structure of the fossa ovalis, thereby providing a certain retention force and preventing the valve prosthesis from moving when a heart compresses. The anchoring structure of the invention changes the traditional anchoring way of clamping the valve leaflets or grasping the valve leaflets without stretching a chordae tendineae or damaging the valve leaflets.

Preferably, the anchoring structure includes a first anchoring member, the first anchoring member is configured to be provided with at least one protruding portion, and the protruding portion is embedded into the fossa ovalis and adhered to an inner wall of the fossa ovalis for the anchoring effect while providing an effective anchoring force for the valve prosthesis.

The protruding portion is preferably of an arc-shaped structure constituted by rod-shaped members, and then there may be one protruding portion. Preferably, the at least one protruding portions comprises at least two protruding portions, each of the protruding portions extends axially, and the plurality of protruding portions are arranged side by side, wherein distributing side by side refers to that the plurality of protruding portions are arranged along a direction of a long axis of the fossa ovalis. The process of forming the rod-shaped members is simple, and the plurality of protruding portions are embedded into the fossa ovalis and adhered to a short axis simultaneously, so as to provide a more stable retention effect.

Preferably, the anchoring structure further includes a second anchoring member, and the second anchoring member is fixed to an atrium wall by a radial acting force to provide a further anchoring force. The second anchoring member is arranged in a right atrium in a form of oversizing, thereby providing a radial acting force to achieve anchoring.

Preferably, the second anchoring member is configured to be equipped with an outward protruding portion, and the outward protruding portion is embedded into the fossa ovalis to provide a further anchoring force, wherein the outward protruding portion is adhered to the inner wall of the fossa ovalis, which may prevent the second anchoring member from displacing.

In order to prevent a softer fossa ovalis from being damaged by a position where the first anchoring member is connected with the second anchoring member, the second anchoring member is connected with the first anchoring member at an upper edge of the protruding portion; the atrium wall of the upper edge of the fossa ovalis is thicker, which may withstand greater compression while also playing a role in further stabilizing the anchoring structure.

Specifically, the second anchoring member is configured to be of a rod-shaped structure with a circle shape or an arc shape, and an extension direction of the second anchoring member is arranged at a pre-set angle with respect to an extension direction of the first anchoring member, wherein the pre-set angle allows forced portions of the second anchoring member to be close to the fossa ovalis so as to avoid squeezing the triangle of Koch and the conducting tissue. Preferably, the anchoring structure further includes at least one connecting portion, the anchoring structure is fixed to the stent body by the connecting portion, and the connecting portion is configured to extend towards the stent body at a position close to the stent body, so that forced portions of the anchoring structure of the invention is close to the fossa ovalis so as to avoid squeezing the triangle of Koch and the conducting tissue while avoiding shielding the coronary sinus and the inferior vena cava at the bottom region of the right atrium.

Preferably, the number of the connecting portion is plural, and the upper edge of the connecting portion extends away from the axis of the stent body. In other words, the upper edge of the connecting portion is attached to the atrium wall, and the plurality of connecting portions may enlarge a contact area of the anchoring structure, thereby providing an enhanced anchoring force. Meanwhile, the plurality of connecting portions may also be used to stabilize the above anchoring structure; preferably, the connecting portion is arranged uniformly along a circumferential direction of the stent body, with a specific number configured according to the anchoring requirements and the difficulty in compressing.

Preferably, the anchoring structure further includes an extending portion, and the extending portion comprises an anchoring needle piercing into the atrium wall or a barb capturing the tissue, so as to exert the further anchoring effect.

Preferably, the extending portion is configured at an end portion away from the stent body and eschews the fossa ovalis, so as to prevent the fossa ovalis from being punctured by the anchoring needle or the barb.

Preferably, the anchoring structure is made from a material with better bio-compatibility, wherein the material with better bio-compatibility facilitates the endothelialization and may help repair the defects of the fossa ovalis such as a congenital patent foramen ovale, an ostium secundum atrial septal defect or an access hole left on the fossa ovalis after a left cardiac intervention operation.

Preferably, a surface of the anchoring structure is further provided with a film layer or a skirt. The film layer or fabrics may be arranged in the form of filming, suturing and so on. The material of the film layer may be selected from materials with better bio-compatibility and facilitates the endothelialization such as PET, PTFE, ePTFE or PU, which may not only protect the native tissue from being scratched by the stent frame but also enlarge the area of the endothelialization, thereby providing assistance for the anchoring. When the surface of the anchoring structure is covered with the skirt, the skirt may be disposed on the connecting portion of the anchoring structure and a part of the meshes should be exposed, so that squeezing and contacting the Koch triangle are avoided while avoiding shielding the coronary sinus and the inferior vena cava.

Compared with the existing technology, the present invention has the following beneficial effects:
First, according to the invention, the anchoring structure is partially attached to a fossa ovalis of an interatrial septum to form a retention force for the anchoring structure by a depression structure of the fossa ovalis so that an anchoring effect on the valve prosthesis is achieved, which changes the traditional anchoring way of clamping the valve leaflets or capturing the valve leaflets, and will not stretch the chordae tendineae or damage the valve leaflets.

Second, the first anchoring member achieves anchoring by way of embedding the protruding portion into the fossa ovalis, the way of forming the protruding portion is simple and easy to achieve, and the enhanced anchoring force is further provided when the anchoring structure further includes the second anchoring member anchored to the atrium wall and when the second anchoring member is configured to be partially embedded into the fossa ovalis; the first anchoring member works together with the second anchoring member, so that the anchoring is firm.

Third, according to the invention, the anchoring structure plays a fixing role by the connecting portion and extends towards the stent body at the position close to the stent body to eschew the conduction tissue, and the main forced portions of the anchoring structure is close to the fossa ovalis, so as to avoid squeezing the Koch triangle and the conduction tissue while preventing the conduction block.

Certainly, any one product for implementing the present invention is unnecessary to achieve all the above advantages at the same time.

### BRIEF DESCRIPTION OF THE DRAWINGS

Fig. 1 is a front structural diagram of a valve prosthesis according to Embodiment 1 of the invention;
Fig. 2 is a side structural diagram of the valve prosthesis according to Embodiment 1 of the invention;
Fig. 3 is a side structural diagram of the valve prosthesis according to Embodiment 2 of the invention;
Fig. 4 is a top structural diagram of the valve prosthesis according to Embodiment 2 of the invention;
Fig. 5 is a front structural diagram of the valve prosthesis according to Embodiment 2 of the invention;
Fig. 6 is a structural diagram of implanting the valve prosthesis at a tricuspid valve annulus according to Embodiment 2 of the invention;
Fig. 7 is a diagram of the anatomical structure of a right atrium.

Reference for numerals: stent body 110; inflow segment 111; outflow segment 113; transition segment 112; anchoring structure 210; second anchoring member 212; extending portion 213; first anchoring member 211; protruding portion 2111; first connecting segment 2112; outward protruding portion 2121; second connecting segment 2122.

### DESCRIPTION OF THE PREFERRED EMBODIMENTS

The invention provides an implantable tricuspid valve prosthesis, which makes full use of the native structure of the fossa ovalis and provides a certain anchoring force by partially embedding the anchoring structure into the fossa ovalis, thereby achieving anchoring the valve prosthesis.

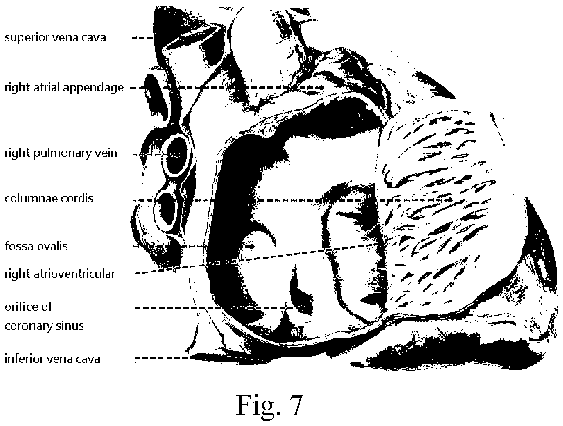

The tricuspid valve located between the right atrium and the right ventricle. The tricuspid valve composite consists of the annulus, the leaflets, the chordae tendineaes, and the papillary muscles, which form a whole functionally and structurally to ensure that blood flows from the atrium to the ventricle. With reference to Fig. 7, a diagram of the anatomical structure of the right atrium is shown, wherein the right atrium has three inlets (i.e., an orifice of superior vena cava, an orifice of inferior vena cava, and an orifice of coronary sinus) and one outlet (i.e., an orifice of tricuspid valve). The orifice of the superior vena cava is located at an upper portion behind the atrium, with the orifice of the inferior vena cava located below. A left front of the orifice of the inferior vena cava is tricuspid valve, and the orifice of coronary sinus is located between the inferior vena cava and the tricuspid valve. An inner sidewall of the right atrium is an interatrial septum, at the bottom of which there is an oval shape depression called fossa ovalis. The fossa ovalis is located at one third of a lower part of the interatrial septum and at an upper left of the inferior vena cava, wherein there is a small trench up to a depth of 3mm to 4mm deep at a central depression thereof.

The native valve annulus is surrounded by the atrioventricular node located below the endocardium of the right side of the interatrial septum. A front inner edge of the coronary sinus, an attachment edge of the septal valve leaflet of the tricuspid valve, and the Tendon of Todaro constitute the Triangle of Koch. An apex of a front of the triangle, i.e., a position where the front valve leaflet is linked with the septal valve leaflet, is the nearby AV node. The AV node is an important part of the heart conducting system. The Tendon of Todaro not only supports and stretches the inferior vena cava valve and the coronary sinus valve, but also supports and fixes the cardiac muscles below the interatrial septum to some extent. Therefore, in the design of replacing the valve with the tricuspid valve in situ, squeezing and covering the Triangle of Koch region should be avoided as much as possible.

The valve prosthesis typically said is composed of two parts: the stent and the prosthetic leaflets fixed thereon, wherein the stent mainly includes a stent body 110 and an anchoring structure 210, the stent body 110 is a hollow column structure with openings at both ends, and the prosthetic leaflets are fixed to an inner circumference of the stent body 110. The stent body 110 is connected with the anchoring structure 210 by riveting, welding, clipping, stitching, and so forth. The anchoring structure 210 may be made from nickel-titanium alloys or other bio-compatible materials with shape memory properties, or from an elastic or malleable deformable material such as a balloon-expandable material.

The valve prosthesis has two states: a compressing state and an expanding state, i.e., both the stent body 110 and the anchoring structure 210 have these two states. If there is no particular emphasis in the present invention, all descriptions should be given for the features in the expanding state.

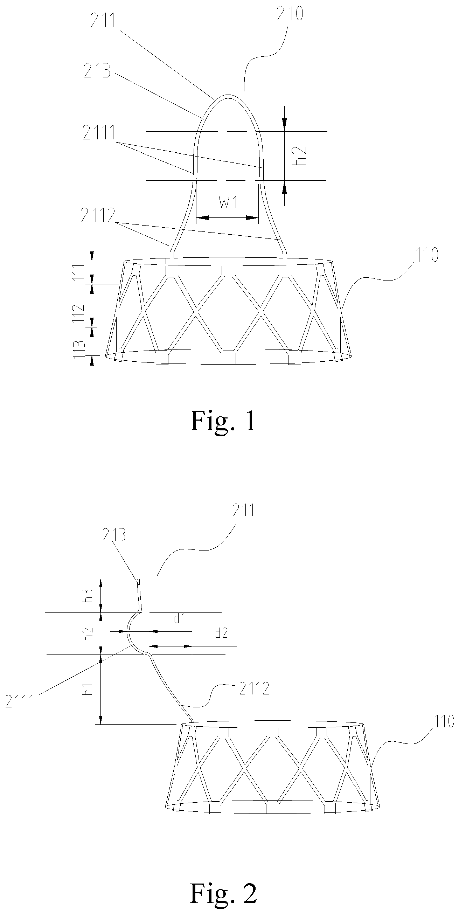

With reference to Fig. 1, the stent body 110 includes an inflow segment 111, an outflow segment 113, and a transition segment 112 located between the inflow segment and the outflow segment. The outflow segment 113 is located downstream of an inflow tract according to the direction of the blood flow. Optionally, the stent body 110 further includes a hanging tab (not shown in Figure), and the hanging tab is connected with an end portion of the outflow segment 113 away from the transition segment 112; the hanging tab is configured to be connected with a delivery system, so as to ensure that while loading the valve in the delivery system, releasing the valve from the delivery system, or delivering the valve through the delivery system in vivo, the relative location between the valve prosthesis and the delivery system remains the same.

The shape of a cross-section of the stent body 110 may be a circle shape, an elliptic shape, a D shape, a flower shape, or other irregular shapes. The stent body 110 may be made from metals such as nitinol, titanium alloy, cobalt chromium alloy, MP35n, SUS316, L605, Phynox/Elgiloy, and platinum chromium, or from other bio-compatible metals known by those skilled in the art. Optionally, the stent body 110 may also be made from an elastic or malleable deformable material such as a balloon-expandable material or a shape memory alloy that may respond to temperature changes to convert between a collapsed state and an expanded state. Preferably, the stent body 110 is made by cutting nickel-titanium alloy tubing, an outer diameter of the tubing ranges from 5mm to 15mm, and the size of the diameter after formation is selected according to actual needs.

The stent body 110 has a significant radial stiffness and a significant axial stiffness, which may withstand the stretch on the valve leaflets. The stent body 110 consists of structure units with a changeable axial morphology such as a mesh structure unit or a wave-shaped structure unit; these structure units are connected with each other in the circumferential direction, consisting of at least one row of structure units in the axial direction, wherein the plurality of rows of units in the axial direction may be directly or indirectly connected with each other. The stent body 110 is preferably made of the mesh structure, wherein the mesh unit is a mesh unit capable of forming an enclosed shape such as a triangle, a diamond, a pentagon, or a droplet shape, and preferably is of a diamond structure.

An inner surface or an outer surface or both two surfaces of the stent body 110 are covered with a skirt to achieve a sealing function, so as to ensure that a single channel of the blood is the outflow segment end flowing from the inflow segment end of the valve leaflets to the prosthesis valve leaflet. The skirt is made from pericardiums (a porcine pericardium, a bovine pericardium, an ovine pericardium, and so forth) or other bio-compatible high polymer materials (e.g., PET (polyethylene terephthalate), PTFE (polytetrafluoro-ethylene), and so forth).

The valve prosthesis includes at least two prosthetic valve leaflets. The number of the prosthetic valve leaflet is the same as or different from that of the native valve leaflets, and is prepared by animal pericardiums or other bio-compatible polymer materials. One end of the valve leaflet is directly or indirectly connected with the stent body 110 stably, and the other end of the valve leaflet is a free end. In the working state, the function of opening and closing the blood channel is realized by replacing the native valve leaflets with prosthetic valve leaflet.

The above-mentioned valve prosthesis is implanted into the heart through the delivery system; the valve prosthesis is loaded into the delivery apparatus such as a sheath after being pressed and held, and released after being implanted to a target position; the released valve prosthesis expands and is anchored to the target position.

The following further describes the present invention in combination with specific embodiments.

In the description of the present invention, it should be noted that orientations or position relationships indicated by terms "center", "upper", "lower", "left", "right", "vertical", "horizontal", "inside", "outside" and the like are orientations or position relationships shown in the drawings, and these terms are merely for facilitating description of the present invention and simplifying the description, but not for indicating or implying that the mentioned device or elements must have a specific orientation and must be established and operated in a specific orientation, and thus, these terms cannot be understood as a limitation to the present invention.

In the description of the present invention, it should be noted that "valve prosthesis" and "valve" have the same meaning. In the description of the invention, it should be noted that the "axial direction" refers to the axial direction of the stent body, and "being above" includes not only "being right above" but also includes "being above the side".

In the description of the present invention, it should be noted that, unless otherwise clearly specified and limited, meanings of terms "install", "connected with", and "connected to" should be understood in a board sense. For example, the connection may be a fixed connection, a removable connection, or an integral connection; may be a mechanical connection or an electrical connection; may be a direct connection or an indirect connection by using an intermediate medium; or may be intercommunication between two components. For those of ordinary skill in the art, specific meanings of the above terms in the present invention may be understood based on specific situations.

As used in the specification, singular forms "a/an," "one," and "the/that" include plural objects, unless otherwise explicitly stated. Terms like "first", "second", "third" etc. are only used for description, not be considered as a designation or designation of relative importance. The term "or" is usually used to include the meaning of "and/or", unless otherwise expressly stated.

### Embodiment 1

The embodiment provides a tricuspid valve prosthesis. The valve prosthesis includes a stent body 110, the stent body 110 being implanted at a tricuspid valve annulus for supporting the prosthetic leaflets; the valve prosthesis further includes an anchoring structure 210 disposed above the stent body 110 for anchoring the stent body 110 at the native valve annulus for preventing the stent body from displacing, wherein the anchoring structure 210 is configured to be partially attached to a fossa ovalis of an interatrial septum to form a retention force by being attached to the fossa ovalis so that an anchoring effect on the valve prosthesis is achieved.

In the embodiment, inspired by the native structure of the fossa ovalis, a new anchoring structure is proposed; the anchoring structure 210 is configured by way of being partially embedded into the fossa ovalis and being attached to the fossa ovalis, and the valve prosthesis is prevented from being displaced in the process of heart compression by the retention force generated by a depression structure of the fossa ovalis.

With reference to Figs. 1 to 2, Fig. 1 is a front structural diagram of a valve prosthesis in the embodiment, and Fig. 2 is a side structural diagram of the valve prosthesis in the embodiment.

The anchoring structure 210 includes a first anchoring member 211, the first anchoring member 211 is configured to be provided with at least one protruding portion 2111, and the protruding portion 2111 is embedded into the fossa ovalis for the anchoring effect. In the embodiment, the first anchoring member 211 is configured to be provided with two protruding portions 2111, wherein the protruding portion 2111 is of a rod-shaped structure with an arc shape and extends along a short axis direction of the fossa ovalis, and the two protruding portions 2111 is of a leftright parallel structure. When being implanted, the protruding portions 2111 are embedded into the fossa ovalis, and the two protruding portions 2111 are arranged along a long axis direction of the fossa ovalis, which may provide a more stable anchoring force as compared with a single protruding portion 2111. Naturally, in other alternative embodiments, there may also be more than two protruding portions 2111, which may be configured according to actual needs.

The anchoring structure 210 further includes a connecting portion, and the connecting portion fixes the anchoring structure 210 above the stent body 110. The connecting portion may be integral with other parts of the anchoring structure 210 (e.g., the protruding portion 2111 in the embodiment) and then be fixed to the stent body 110 by way of welding or stitching or so; or the connecting portion may also be connected with other parts of the anchoring structure 210 and the stent body 110 by way of tethers, stitching and knotting.

In the embodiment, the first anchoring member 211 is formed by bending a rod-shaped member, the first anchoring member 211 bends to form a symmetrical structure at an intermediate position and protrudes outwards at a pre-set position away from the stent body 110, and two above protruding portions 2111 are formed on the two left and right rod-shaped structures respectively; a first connecting segment 2112 is formed at an end close to the stent body 110, the two first connecting segments 2112 are located at a lower edge of the protruding portion 2111 respectively, and the first connecting segment 2112 is the connecting portion of the anchoring structure 210 in the embodiment. When being implanted, a junction of the first connecting segment 2112 and the protruding portion 2111 is adhered to a lower edge of the fossa ovalis. In the embodiment, the way of integrally forming the first anchoring member 211 has the advantages of simple structure and is easy to implement; and the formed first anchoring member 211 is of a smooth and rounded structure, which will not cause damages to the native tissue. Meanwhile, compared with the single first connecting segment 2112, the protruding portions 2111 are connected to the stent body 110 by the first connecting segment 2112 respectively, which may improve the stability of the structure of the first anchoring member 211 and prevent twisting and deformation. Naturally, in other embodiments, the first connecting segment 2112 and the protruding portion 2111 may be formed by being manufactured independently first and connected with each other then.

In a direction from the protruding portion 2111 to the stent body 110, the first connecting segment 2112 extends towards the stent body 110 gradually. The first connecting segment 2112 is located at a lower edge of the first anchoring member 211. Since a distance between the orifice of coronary sinus and the orifice of tricuspid valve is very short and the orifice of coronary sinus cannot be shielded, a lower end of the first anchoring member 211, i.e., the orifice of coronary sinus, should be configured to be away from the atrium wall to leave an enough space for preventing the orifice of coronary sinus from being shielded.

In the embodiment, the protruding portion 2111 is configured by way of being capable of being embedded into the fossa ovalis and adhered to the short axis of the fossa ovalis, so as to provide the effective anchoring force. A maximum depth d1 of the protruding portion 2111 ranges from 2mm to 4mm, and a height h2 thereof ranges from 6mm to 10mm, wherein if the maximum depth or the height is too large, the resistant is too large, and there is a risk of destroying the native structure of the fossa ovalis; if the maximum depth or the height is too small, the protruding portion cannot be well embedded in the fossa ovalis, and it is difficult to provide a stable anchoring force. Here, the "depth" refers to a vertical distance from a tangent of an outer surface of the protruding portion 2111 to the lower edge of the protruding portion 2111, and the "height" refers to a size along the axial direction of the stent body 110.

A distance w1 between lower edges of two protruding portions 2111 ranges from 5mm to 15mm, and a suitable distance is configured between the protruding portions 2111 for being embedded into the fossa ovalis so as to provide the stable anchoring force. A depth d2 (i.e., the vertical distance) between an upper edge of the first connecting segment 2112 and the lower edge of the first connecting segment 2112 ranges from 2mm to 9mm, a height h1 of the first connecting segment 2112 ranges from 11mm to 13mm, and the first connecting segment 2112 will abut the protruding portion 2111 against the fossa ovalis to generate the above anchoring force. Two first connecting segments 2112 are arranged along two sides of a trapezoid substantially so that the structure of the first anchoring member 211 is more firm, and widths of the lower edges of the two first connecting segments 2112 are related to nodes of the stent body 110.

In some embodiments, the first anchoring member 211 further includes an extending portion 213, and the extending portion 213 includes an anchoring needle piercing into the atrium wall. In the embodiment, the extending portion 213 is configured when the first anchoring member 211 is being formed, and the extending portion 213 is located at a free end of the first anchoring member 211; when being implanted, the extending portion 213 protrudes out of an upper edge of the fossa ovalis to enhance the anchoring of the valve prosthesis by piercing the anchoring needle into the atrium wall. On the other hand, an upper part and a lower part of the protruding portion 2111 is forced simultaneously, so as to further stabilize the protruding portion 2111 into the fossa ovalis. In other embodiments, the extending portion 213 may also be a barb for capturing the tissue, which similarly further plays the anchoring role.

Further, a surface of the anchoring structure 210 is further provided with a film layer or a skirt. In some embodiments, a surface of the protruding portion 2111 is further provided with the film layer. The film layer is made from the high-polymer material, which may be arranged in the form of a film, weaving woven fabrics, and so on. Specifically, the high-polymer material may be selected from materials with better bio-compatibility and facilitates the endothelialization such as PET, PTFE, ePTFE or PU, which may not only protect the native tissue from being scratched by the stent frame but also may enlarge the area of the endothelialization, thereby providing assistance for the anchoring. In some embodiments, a surface of the first connecting segment 2112 is partially covered with the skirt or not covered with the skirt, with at least a part of large meshes exposed, which, with cooperation of the design of large mesh or the design of notch of the stent body 110, may avoid squeezing and contacting the Koch triangle while avoiding shielding the orifice of coronary sinus and the orifice of inferior vena cava. In some embodiments, the extending portion 213 is covered with the skirt, and the skirt is the anchoring needle while protecting the native tissue from being scratched by the stent frame.

In an optional embodiment, the anchoring structure 210 is further provided with a fixing tab, and the fixing tab is disposed on the extending portion 213. The hanging tab is configured to be connected with a delivery system, so as to ensure that while loading the valve in the delivery system, releasing the valve from the delivery system, or delivering the valve through the delivery system in vivo, the relative location between the valve prosthesis and the delivery system remains the same.

In the embodiment, the above rod-shaped member may have a certain width, and the width of the rod-shaped member determines the contact area with the native tissue, i.e., different anchoring forces may be provided by adjusting the width of the rod-shaped member.

In the embodiment, the positions and the effects when the valve prosthesis is placed as a whole are:
in the embodiment, the inflow segment of the stent body 110 is placed at the native valve annulus, a bit of oversizing of the outflow segment may open the native valve leaflets to prevent the prosthesis valve from being affected by the free movement of the native valve leaflets, and the first connecting segment 2112 is adhered to the right atrium wall or suspended in the right atrium; the protruding portion 2111 is embedded into the fossa ovalis, the junction of the first connecting segment 2112 and the protruding portion 2111 is adhered to a lower edge of the fossa ovalis, and the extending portion 213 is placed on the upper edge of the fossa ovalis, so as to prevent the prosthesis valve from displacing after being implanted by using the depression of the fossa ovalis.

The triangle region among the anterior edge of the coronary sinus, the attachment edge of the septal leaflet of the tricuspid valve and the Todaro tendon is called the Triangle of Koch. Excessive stimulation in this triangle region may cause arrhythmia. In the embodiment, the main forced parts of the anchoring structure 210 are near the fossa ovalis, so as to avoid squeezing the Koch triangle and the conducting tissue.

### Embodiment 2

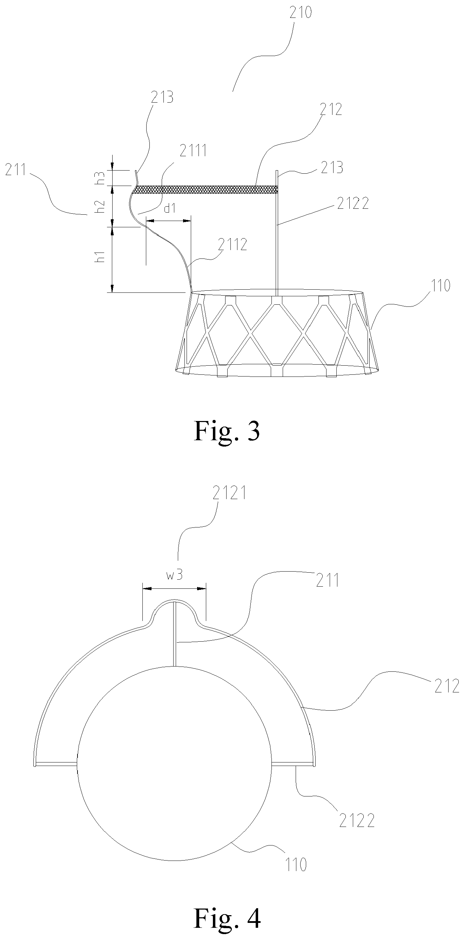

The embodiment provides a tricuspid valve prosthesis, which is an improvement based on Embodiment 1, wherein the anchoring structure 210 further includes a second anchoring member 212, and with reference to Figs. 3 to 6, the second anchoring member 212 is fixed to the atrium wall by a radial acting force, thereby providing an enhanced anchoring force for the valve prosthesis.

With reference to Figs. 3 and 4, in the embodiment, the first anchoring member 211 is of a rod-shaped structure, which is configured with one protruding portion 2111 along the axial direction. The second anchoring member 212 is configured at an upper edge of the protruding portion 2111, and the second anchoring member 212 is configured to be provided with an outward protruding portion 2121. When being implanted, the outward protruding portion 2121 is embedded into the fossa ovalis from the upper edge of the protruding portion 2111 to provide the enhanced anchoring effect, while the forced points being at the upper edge of the fossa ovalis may prevent the native structure of the fossa ovalis from being damaged.

Specifically, the second anchoring member 212 is of the rod-shaped structure with a circle shape or a partial arc shape, and the second anchoring member 212 is disposed by way of being substantially at a right angle with respect to an extension direction of the first anchoring member 211, i.e., the second anchoring member 212 is substantially parallel to an upper end surface of the stent body 110, so that the forced parts of the second anchoring member 212 are near the fossa ovalis to avoid squeezing the Koch triangle and the conduction tissue. The second anchoring member 212 has a chord length w2 ranging from 48mm to 55mm, and is adhered to the ventricle wall to enhance the anchoring strength by way of the overall Oversize. The outward protruding portion 2121 has an arc shape with the chord length ranging from 9mm to 12mm. When being implanted, the outward protruding portion 2121 is adhered to the upper edge of the fossa ovalis. Here, the "chord length" refers to a distance between two furthest endpoints of the arc shape.

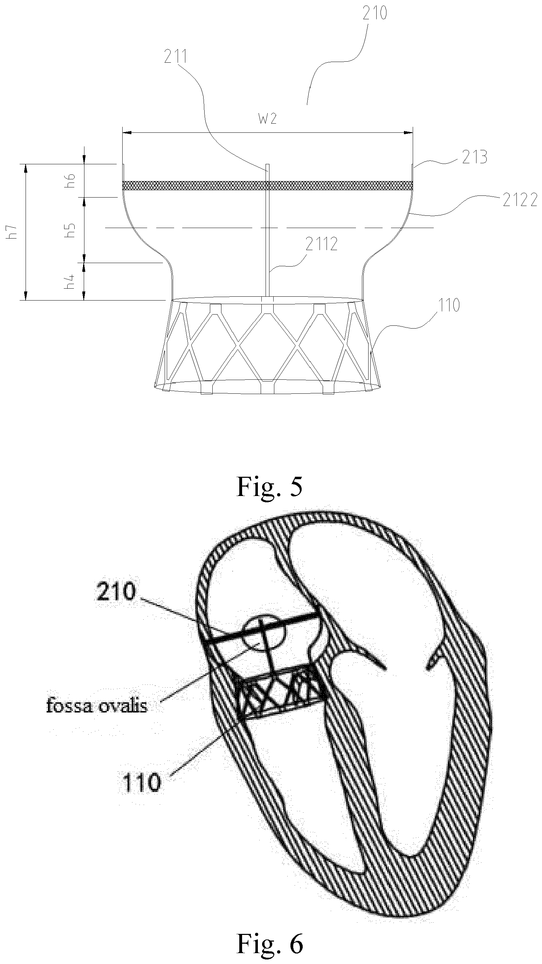

With reference to Fig. 5, the second anchoring member 212 is configured to be of the partial arc shape, the connecting portion of the anchoring structure 210 further includes a second connecting segment 2122 disposed at two end portions of the second anchoring member 212, and the second connecting segment 2122 fixes the second anchoring member 212 to the stent body 110. The second connecting segment 2122 has a height h7 ranging from 20mm to 24mm, and a width of a junction between the second connecting segment and the stent body 110 is related to the nodes of the stent body 110. The second connecting segment 2122 is connected with the second anchoring member 212 by way of stitching, welding and so on. The second connecting segment 2122 is divided into an upper part, a middle part and a lower part, wherein the upper part is adhered to the atrium wall, with a height h6 ranging from 3mm to 6mm; the middle part is a transition portion, and provides a guarantee for the upper part of the second connecting segment 2122 to be able to be adhered to the atrium wall, with a height h5 ranging from 9mm to 13mm; the lower part is connected with the stent body 110. The lower part of the second connecting segment 2122 extends towards the stent body 110, i.e., the lower part converges at a position close to the stent body 110, so as to avoid shielding the orifice of coronary sinus and the orifice of inferior vena cava at the bottom region of the right atrium. Naturally, in other embodiments, the number of the second connecting segment 2122 may be one, two or plural, which is designed according to comprehensive consideration of the anchoring requirements and the difficulty in pressing and holding. Preferably, the anchoring structure 210 is of asymmetrical structure, the second connecting segment 2122 is arranged on two sides of the first anchoring member 211 symmetrically or the second connecting segment 2122 is arranged uniformly along the stent body 110 in the circumferential direction, and the number and the connecting positions of the second connecting segment 2122 should be configured according to the anchoring requirements and the difficulty in pressing and holding.

In the embodiment, the second anchoring member 212 is formed to be of the rod-shaped structure with the partial arc shape. In other alternative embodiments, there may be a plurality of the second anchoring members 212, and the plurality of the second anchoring members 212 are connected with each other to form a three-dimensional structure, so that a further stable anchoring force may be provided by the radial acting force of the three-dimensional structure.

In the embodiment, the second anchoring member 212 is configured at the upper edge of the protruding portion 2111. In order to prevent the softer fossa ovalis from being damaged by the junction between the first anchoring member 211 and the second anchoring member 212, the second anchoring member 212 is further connected with the first anchoring member 211 at a pre-set position of the upper edge of the protruding portion 2111 by way of, e.g., welding, stitching, the film layer and so on. The atrium wall of the upper edge of the fossa ovalis is thicker, which may withstand a greater compression while playing a role of further stabilizing the anchoring structure 210.

In the embodiment, the upper part of the second connecting segment 2122 is just one extending portion 213, which may be configured with the anchoring needle, and anchoring the valve prosthesis is enhanced by piercing the atrium wall with the anchoring needle.

With reference to Fig. 6, in the embodiment, the second anchoring member 212 is positioned at the fossa ovalis by using the Oversize and the native anatomical structure. When being implanted, the upper part of the second connecting segment 2122 is adhered to the atrium wall, and the outward protruding portion 2121 is embedded into the fossa ovalis, so as to provide the enhanced anchoring effect and to make the anchoring firm.

Inspired by the native structure of the fossa ovalis, the invention clamps and fixes the anchoring structure in the fossa ovalis partially by making full use of a depression structure of the fossa ovalis, thereby providing a certain retention force and preventing the valve prosthesis from displacing when a heart compresses. The anchoring structure of the invention changes the traditional anchoring way of clamping the valve leaflets or grasping the valve leaflets without stretching a chordae tendineae or damaging the valve leaflets.

The above disclosure is only the preferred embodiment of the present invention. The preferred embodiments do not describe all the details, and are not intended to limit the invention only to be the specific embodiments. It should be understood that these embodiments are only used for illustrating the present invention, but not for the limitation of the scope of the present invention.

It is obvious that various modifications and changes can be made to the content of the specification. The present invention selects and specifically describe the embodiments with the purpose of better explain the principle and practical use of the present invention, such that a person skilled in the art can well utilize the present invention.

## Claims

1. A tricuspid valve prosthesis, comprising:
a stent body (110), implanted at a tricuspid valve annulus for supporting the prosthetic valve leaflets; and
an anchoring structure (210), disposed above the stent body for anchoring the stent body at the native valve annulus for preventing the stent body from displacing;
wherein the anchoring structure (210) is configured to be partially attached to the fossa ovalis of the interatrial septum to form a retention force by being attached to the fossa ovalis so that the anchoring effect on the valve prosthesis is achieved;
wherein the anchoring structure (210) comprises a first anchoring member (211) configured to be provided with at least one protruding portion (2111), and the protruding portion (2111) is embedded into the fossa ovalis for the anchoring effect.

2. The tricuspid valve prosthesis according to claim 1, wherein the at least one protruding portions (2111) comprises at least two protruding portions (2111), each of the protruding portions (2111) extends axially, and the plurality of protruding portions (2111) are arranged side by side.

3. The tricuspid valve prosthesis according to claim 1, wherein the anchoring structure (210) further comprises a second anchoring member (212), and the second anchoring member (212) is fixed to an atrium wall by a radial acting force to provide an enhanced anchoring force.

4. The tricuspid valve prosthesis according to claim 3, wherein the second anchoring member (212) is configured to be equipped with an outward protruding portion (2121), and the outward protruding portion (2121) is embedded into the fossa ovalis to provide a further anchoring force.

5. The tricuspid valve prosthesis according to claim 3, wherein at an upper edge of the protruding portion (2111), the second anchoring member (212) is connected with the first anchoring member (211).

6. The tricuspid valve prosthesis according to claim 3, wherein an extension direction of the second anchoring member (212) is arranged at a pre-set angle with respect to an extension direction of the first anchoring member (211), wherein the pre-set angle allows forced portions of the second anchoring member to be close to the fossa ovalis so as to avoid squeezing the triangle of Koch and the conducting tissue.

7. The tricuspid valve prosthesis according to claim 1, wherein the anchoring structure (210) further comprises at least one connecting portion, the anchoring structure (210) is fixed to the stent body by the connecting portion, and the connecting portion is configured to extend towards the stent body (110) at a position close to the stent body (110).

8. The tricuspid valve prosthesis according to claim 7, wherein the number of the connecting portion is plural, and an upper edge of the connecting portion extends away from an axis of the stent body (110).

9. The tricuspid valve prosthesis according to claim 1, wherein the anchoring structure (210) further comprises an extending portion (213), and the extending portion (213) comprises an anchoring needle piercing into the atrium wall or a barb capturing a tissue.

10. The tricuspid valve prosthesis according to claim 9, wherein the extending portion (213) is configured at an end portion away from the stent body (110).

11. The tricuspid valve prosthesis according to claim 1, 2 or any one of claims 4 to 10, wherein a surface of the anchoring structure (210) is further provided with a film layer or a skirt.

## Patentansprüche

1. Trikuspidalklappenprothese, umfassend:
- einen Stentkörper (110), der an einem Trikuspidalklappenanulus implantiert ist, um die prothetischen Klappensegel zu stützen; und
- eine Verankerungsstruktur (210), die oberhalb des Stentkörpers zur Verankerung des Stentkörpers am nativen Ventilanulus angeordnet ist, um eine Verschiebung des Stentkörpers zu verhindern;
- wobei die Verankerungsstruktur (210) so eingerichtet ist, dass sie teilweise an der Fossa ovalis des interatrialen Septums befestigt ist, um eine Haltekraft zu bilden, indem sie an der Fossa ovalis befestigt wird, so dass die Verankerungswirkung auf die Klappenprothese erreicht wird;
- wobei die Verankerungsstruktur (210) ein erstes Verankerungselement (211) umfasst, das so eingerichtet ist, dass es mit wenigstens einem vorstehenden Abschnitt (2111) versehen ist, und der vorstehende Abschnitt (2111) für den Verankerungseffekt in die Fossa ovalis eingebettet ist.

2. Trikuspidalklappenprothese nach Anspruch 1, wobei die wenigstens einen vorstehenden Abschnitte (2111) wenigstens zwei vorstehende Abschnitte (2111) umfassen, jeder der vorstehenden Abschnitte (2111) sich axial erstreckt, und die mehreren vorstehenden Abschnitte (2111) nebeneinander angeordnet sind.

3. Trikuspidalklappenprothese nach Anspruch 1, wobei die Verankerungsstruktur (210) ferner ein zweites Verankerungselement (212) umfasst und das zweite Verankerungselement (212) durch eine radial wirkende Kraft an einer Vorhofwand fixiert ist, um eine verbesserte Verankerungskraft zu bieten.

4. Trikuspidalklappenprothese nach Anspruch 3, wobei das zweite Verankerungselement (212) so ausgebildet ist, dass es mit einem nach außen vorstehenden Teil (2121) versehen ist, und der nach außen vorstehende Teil (2121) in die Fossa ovalis eingebettet ist, um für eine weitere Verankerungskraft zu sorgen.

5. Trikuspidalklappenprothese nach Anspruch 3, wobei an einer Oberkante des vorstehenden Abschnitts (2111) das zweite Verankerungselement (212) mit dem ersten Verankerungselement (211) verbunden ist.

6. Trikuspidalklappenprothese nach Anspruch 3, wobei eine Verlängerungsrichtung des zweiten Verankerungselements (212) in einem voreingestellten Winkel in Bezug auf eine Verlängerungsrichtung des ersten Verankerungselements (211) angeordnet ist, wobei der voreingestellte Winkel es ermöglicht, dass erzwungene Abschnitte des zweiten Verankerungselements nahe an der Fossa ovalis sind, um eine Quetschung des Koch-Dreiecks und des leitenden Gewebes zu vermeiden.

7. Trikuspidalklappenprothese nach Anspruch 1, wobei die Verankerungsstruktur (210) ferner wenigstens einen Verbindungsabschnitt umfasst, die Verankerungsstruktur (210) durch den Verbindungsabschnitt am Stentkörper befestigt ist, und der Verbindungsabschnitt so eingerichtet ist, dass er sich in einer Position nahe am Stentkörper (110) zum Stentkörper (110) erstreckt.

8. Trikuspidalklappenprothese nach Anspruch 7, wobei die Zahl des Verbindungsabschnitts mehrfach ist und sich eine Oberkante des Verbindungsabschnitts von einer Achse des Stentkörpers (110) weg erstreckt.

9. Trikuspidalklappenprothese nach Anspruch 1, wobei die Verankerungsstruktur (210) ferner einen verlängerten Abschnitt (213) und der verlängerte Abschnitt (213) eine in die Vorhofwand eindringende Verankerungsnadel oder einen ein Gewebe erfassenden Widerhaken umfasst.

10. Trikuspidalklappenprothese nach Anspruch 9, wobei der verlängerte Abschnitt (213) an einem vom Stentkörper (110) entfernten Endabschnitt eingerichtet ist.

11. Trikuspidalklappenprothese nach Anspruch 1, 2 oder einem der Ansprüche 4 bis 10, wobei eine Oberfläche der Verankerungsstruktur (210) ferner mit einer Filmschicht oder einer Schürze versehen ist.

## Revendications

1. Une prothèse valvulaire tricuspide, comprenant :
un corps d'endoprothèse (110), implanté au niveau d'un anneau valvulaire tricuspide pour supporter les prothèses de feuillets valvulaires ; et
une structure d'ancrage (210), disposée au-dessus du corps d'endoprothèse pour ancrer le corps d'endoprothèse au niveau de l'anneau valvulaire natif afin d'empêcher le corps d'endoprothèse de se déplacer ;
dans laquelle la structure d'ancrage (210) est configurée pour se fixer partiellement à la fosse ovale du septum interatrial pour former une force de rétention au moyen de la fixation à la fosse ovale, de manière à obtenir l'effet d'ancrage sur la prothèse valvulaire ;
dans laquelle la structure d'ancrage (210) comprend un premier élément d'ancrage (211) configuré pour comprendre au moins une partie en saillie (2111), et la partie en saillie (2111) est incorporée dans la fosse ovale pour l'effet d'ancrage.

2. La prothèse valvulaire tricuspide selon la revendication 1, dans laquelle l'au moins une partie en saillie (2111) comprend au moins deux parties en saillie (2111), chacune des parties en saillie (2111) s'étendant de manière axiale, et la pluralité des parties en saillie (2111) sont agencées côte à côte.

3. La prothèse valvulaire tricuspide selon la revendication 1, dans laquelle la structure d'ancrage (210) comprend en outre un deuxième élément d'ancrage (212), et le deuxième élément d'ancrage (212) est fixé à une paroi d'oreillette par une force d'action radiale pour fournir une force d'ancrage améliorée.

4. La prothèse valvulaire tricuspide selon la revendication 3, dans laquelle le deuxième élément d'ancrage (212) est configuré pour être équipé d'une partie en saillie vers l'extérieur (2121), et la partie en saillie vers l'extérieur (2121) est intégrée dans la fosse ovale pour fournir une force d'ancrage supplémentaire.

5. La prothèse valvulaire tricuspide selon la revendication 3, dans laquelle au niveau d'un bord supérieur de la partie en saillie (2111), le deuxième élément d'ancrage (212) est connecté au premier élément d'ancrage (211).

6. La prothèse valvulaire tricuspide selon la revendication 3, dans laquelle une direction d'extension du deuxième élément d'ancrage (212) est agencée au niveau d'un angle prédéfini par rapport à une direction d'extension du premier élément d'ancrage (211), dans laquelle l'angle prédéfini permet aux parties forcées du deuxième élément d'ancrage d'être fermées à la fosse ovale de façon à éviter de presser le triangle de Koch et le tissu conducteur.

7. La prothèse valvulaire tricuspide selon la revendication 1, dans laquelle la structure d'ancrage (210) comprend en outre au moins une partie de liaison, la structure d'ancrage (210) est fixée au corps d'endoprothèse par la partie de liaison, et la partie de liaison est configurée pour s'étendre vers le corps d'endoprothèse (110) au niveau d'une position proche du corps d'endoprothèse (110).

8. La prothèse valvulaire tricuspide selon la revendication 7, dans laquelle le nombre des parties de liaison est pluriel, et un bord supérieur de la partie de liaison s'étend à distance d'un axe du corps d'endoprothèse (110).

9. La prothèse valvulaire tricuspide selon la revendication 1, dans laquelle la structure d'ancrage (210) comprend en outre une partie d'extension (213), et la partie d'extension (213) comprend un perçage d'aiguille d'ancrage dans la paroi d'oreillette ou un ardillon capturant un tissu.

10. La prothèse valvulaire tricuspide selon la revendication 9, dans laquelle la partie d'extension (213) est configurée au niveau d'une partie d'extrémité à distance du corps d'endoprothèse (110).

11. La prothèse valvulaire tricuspide selon les revendications 1, 2 ou selon l'une quelconque des revendications 4 à 10, dans laquelle une surface de la structure d'ancrage (210) comprend en outre une couche de film ou une jupe.
